# EUROPÄISCHE PATENTSCHRIFT

(11) **EP 1 420 694 B1**
(45) Veröffentlichungstag und Bekanntmachung des Hinweises auf die Patenterteilung: **24.08.2011**
(21) Anmeldenummer: 02790166.9
(22) Anmeldetag: 17.07.2002
(51) Int. Cl.: A61B 5/15, A61B 5/00, B01L 3/00

(54) **SYSTEM ZUR ENTNAHME KLEINER KÖRPERFLÜSSIGKEITSMENGEN**
SYSTEM FOR COLLECTING SMALL AMOUNTS OF BODY FLUID
SYSTEME DE PRELEVEMENT DE LIQUIDES ORGANIQUES EN PETITES QUANTITES

(30) Priorität: 20.07.2001 DE 10134650
(43) Veröffentlichungstag der Anmeldung: 26.05.2004
(73) Patentinhaber: Roche Diagnostics GmbH, 68305 Mannheim (DE); F.Hoffmann-La Roche AG, 4070 Basel (CH)
(72) Erfinder: EFFENHAUSER, Carlo, 69469 Weinheim (DE); HEIN, Heinz-Michael, 64295 Darmstadt (DE); KOELKER, Karl-Heinz, 67269 Gruenstadt (DE); DECK, Frank, 67150 Niederkirchen (DE)
(86) Internationale Anmeldenummer: PCT/EP2002/008017
(87) Internationale Veröffentlichungsnummer: WO 2003/009759

(56) Entgegenhaltungen:
- WO-A-99/30158
- WO-A2-95/10223
- WO-A2-95/10223
- DE-A- 19 753 847
- US-A- 4 637 403
- US-A- 5 192 502
- US-A- 5 700 695
- US-A- 6 099 484

## Beschreibung

Die vorliegende Erfindung fällt in das Gebiet der Analyse von Körperflüssigkeiten zur Erstellung einer Diagnose bzw. zur Überwachung der Konzentration von Stoffwechselparametern, wie zum Beispiel der Blutglukosekonzentration.

Die Erfindung betrifft ein System zur Entnahme kleiner Körperflüssigkeitsmengen beinhaltend eine Antriebseinheit mit einer Halterung zur Aufnahme einer disposiblen Stecheinheit, welche einen Haltebereich und eine mit dem Haltebereich verbundene Kapillarstruktur aufweist. Die Kapillarstruktur hat mindestens einen Kapillarkanal sowie eine von der Halterung abgewandte Spitze zum Einstechen in die Haut. Die Kapillarstruktur ist zumindest in einem Teil ihrer Längsausdehnung nach außen offen.

Im Stand der Technik sind bereits Systeme zur Entnahme von Körperflüssigkeiten bekannt, bei denen die Körperflüssigkeit in ein disposibles Element aufgenommen wird. Aus dem Dokument EP 0 199 484 sind Blutentnahme und Analysesysteme bekannt, die eine disposible Einheit mit einer Kapillare zur Aufnahme von Körperflüssigkeit und zum Transportieren der Körperflüssigkeit in einen Detektionsbereich aufweisen. Eine Weiterentwicklung dieses Konzeptes ist in der WO 97/42888 beschrieben. Die hier beschriebene Anordnung ist insbesondere zur Aufnahme relativ kleiner Körperflüssigkeitsmengen geeignet, welche primär durch den Andruck eines Ringes auf den Umgebungsbereich einer Entnahmestelle und einer Pumpbewegung erfolgt. Aus der EP 0 723 418 ist ein System zur Analyse auf Basis kleiner Mengen an interstitieller Flüssigkeit bekannt. Hierzu wird eine sehr dünne, geschlossene Kanüle in die Dermis eingeführt und durch Ausüben eines Druckes auf den die Einstichstelle umgebenden Bereich interstitielle Flüssigkeit durch die Kanüle auf eine Testzone befördert. In einem Ausführungsbeispiel (Fig.26-28) ist eine Stecheinheit beschrieben, die im Bereich der Spitze einen nach außen offenen Kapillarkanal aufweist. Eine stark miniaturisierte Anordnung, die ebenfalls mit einer geschlossenen Kanüle zur Aufnahme von Körperflüssigkeit arbeitet, ist aus US 5,801,057 bekannt. Ein besonderer Vorzug dieser Anordnung besteht in der äußerst dünnen Kanüle, welche zumindest im Armbereich eines Patienten weitestgehend schmerzfrei eingestochen werden kann.

Während die in US 5,801,057 beschriebene Anordnung bereits eine Vielzahl der in der Praxis bestehenden Anforderungen erfüllt, so sind doch einige Eigenschaften verbesserungsfähig. Ein generelles Problem, das Entnahmevorrichtungen gemäß dem vorstehend genannten Dokument zugrunde liegt, ist es, die verwendeten Kanülen kostengünstig und möglichst klein herzustellen. Insbesondere bei einer Miniaturisierung, die im Hinblick auf einen möglichst geringen Ein-stichschmerz und eine möglichst kleine Einstichwunde wünschenswert ist, treten hohe Herstellungskosten für die sehr dünnen geschlossenen Kanülen auf und es stellen sich prinzipielle Machbarkeitsprobleme.

Aus US-A-5 700 695 ist eine Hohlnadel mit einer sich axial erstreckenden Aussparung am distalen, eine Spitze aufweisenden Ende bekannt. Die Aussparung bewirkt eine effektivere Probennahme aus menschlichem Gewebe, da sie im wesentlichen ein Verstopfen des Nadellumens verhindert. Die Oberfläche des Lumens ist hydrophob, so dass eine negative Kapillarkraft vorhanden ist. Daher ist eine thermische Druckkamer erforderlich um eine Probe durch das Lumen einzuziehen.

Gemäß der vorliegenden Erfindung werden die Anforderungen an Systeme zur Entnahme kleiner Körperflüssigkeitsmengen insbesondere dadurch gelöst, dass statt der im Stand der Technik bekannten geschlossenen Kanülenstrukturen Kanülen mit einer offenen Kapillarstruktur verwendet werden. Hierdurch ergeben sich nicht nur erhebliche Vorteile in der Herstellung, die die Entnahmesysteme kostengünstiger und einfacher herstellbar machen, sondern es ergeben sich auch gravierende Vorteile bei der Aufnahme von Körperflüssigkeit, da diese nicht nur wie bei geschlossenen Kanülenstrukturen, über die Kanülenspitze eintreten kann, sondern auch über den offenen Bereich der Kapillarstruktur. Darüber hinaus kann man auch den Vorteil ausnutzen, dass der offene Kanalbereich der Nadel auch in noch eingestochenem Zustand in der Lage ist, Blut von der Hautoberfläche aufzunehmen und zu einer Nachweiszone zu transportieren.

Systeme zur Entnahme kleiner Körperflüssigkeitsmengen haben ihren Anwendungsbereich insbesondere im sogenannten Spot-monitoring, bei dem die Konzentration von bestimmten, in Körperflüssigkeiten enthaltenen Analyten zu einem gegebenen Zeitpunkt bestimmt wird. Derartige Messungen können in zeitlichen Abständen wiederholt erfolgen, um eine Veränderung der Analytkonzentration zu überwachen. Eine solche Analyse unter Verwendung disposibler Testelemente hat sich insbesondere im Bereich der Blutzuckermessung von Diabetikern als sehr vorteilhaft erwiesen. Treten bei einem Diabetiker über einen gewissen Zeitraum zu hohe Blutzuckerwerte auf (Hyperglykämie), so kann dies gravierende Langzeitschäden wie Erblindungen und Gangräne zur Folge haben. Gerät ein Diabetiker hingegen in einen Zustand der Unterzuckerung (Hypoglykämie), weil er beispielsweise eine zu große Dosis Insulin gespritzt hat, so kann dies lebensbedrohend werden, wenn der Diabetiker in einen sogenannten hypoglykämischen Schock fällt. Durch eine regelmäßige Kontrolle des Blutzuckerspiegels kann der Diabetiker hingegen hyper- und hypoglykämische Zustände vermeiden bzw. auch dauerhaft lernen, Essverhalten, körperliche Betätigung und Insulinmedikation aufeinander abzustellen. Neben einer Verbesserung bzw. Erhaltung der gesundheitlichen Situation von Diabetikern bringt eine regelmäßige Blutzuckerkontrolle auch erhebliche gesamtwirtschaftliche Vorteile, da hohe Kosten für Folgeerkrankungen vermieden werden können. Gründe, die einer noch weiteren Verbreitung und konsequenten Anwendung der Blutzuckerkontrolle entgegenstehen, sind in erster Linie der Schmerz für die notwendige Entnahme von Körperflüssigkeit und die bei im Markt verbreiteten Systemen umfangreichen Handhabungsschritte. Bei heute verbreiteten Systemen muss der Diabetiker oder das medizinisches Personal zunächst einen Blutstropfen gewinnen, was meistens an der Fingerbeere erfolgt. Soll dies möglichst schmerzfrei erfolgen, so werden sogenannte Stechhilfen eingesetzt. Eine Stechhilfe muss zunächst mit einer Lanzette bestückt, gespannt, auf die Körperoberfläche aufgesetzt und ausgelöst werden. Nach dem Stichvorgang muss der Benutzer seinen Finger melken, um einen Blutstropfen aus der Einstichwunde, welche ja möglichst klein sein soll, herauszubefördern. Bereits vor dieser Prozedur muss der Diabetiker ein Blutzuckermessgerät mit einem Teststreifen bestücken und in Betrieb nehmen. Der Blutstropfen kann nunmehr auf den Teststreifen aufgegeben werden und nach beispielsweise 10 Sekunden steht ein Blutzuckermesswert zur Verfügung. Der Benutzer muss nunmehr noch die benutzte Lanzette und den benutzten Teststreifen entsorgen. Die vorliegende Erfindung ermöglicht es, den beschriebenen Blutzuckermessvorgang stark zu vereinfachen, indem ein sogenanntes integriertes System zur Verfügung gestellt wird, bei dem Stechen, Probennahme und analytische Reaktion mit derselben (disposiblen) Einheit erfolgen.

Ein System gemäß der vorliegenden Erfindung dient zur Entnahme kleiner Mengen an Körperflüssigkeit. Unter Körperflüssigkeiten werden in diesem Zusammenhang insbesondere Blut, interstitielle Flüssigkeit und Gemische dieser Körperflüssigkeiten verstanden. Während bei herkömmlichen Systemen zur Blutentnahme diese im Regelfall an der Fingerbeere erfolgt, können mit dem erfindungsgemäßen Entnahmesystem Körperflüssigkeiten auch an anderen Körperstellen, wie zum Beispiel dem Unterarm entnommen werden.

Eine disposible Stecheinheit zur Entnahme kleiner Körperflüssigkeitsmengen gemäß der vorliegenden Erfindung besitzt einen Haltebereich, mit dem das proximale Ende einer länglichen Kapillarstruktur mit mindestens einem Kapillarkanal zum Transport von Körperflüssigkeit verbunden ist. Das distale Ende der Kapillarstruktur ist geeignet, Haut zu durchstechen und entlang seiner Längsausdehnung ist zumindest ein Teil der Kapillarstruktur nach außen offen. Unter einer Kapillarstruktur wird im Rahmen der Erfindung ein Körper verstanden, der, wenn er mit seinem distalen Bereich in Kontakt mit Körperflüssigkeit gebracht wird, diese in Folge von Kapillarkräften in Richtung des proximalen Endes der Kapillarstruktur transportiert. Betreffend dieser Funktion ist die erfindungsgemäße Kapillarstruktur ähnlich den in US 5,801,057 und EP 0 723 418 beschriebenen Hohlkanülen. Ein wesentlicher Unterschied ist jedoch dadurch gegeben, dass zumindest ein Bereich, der zumindest einen Teil der Längsausdehnung der Kapillarstruktur umfasst, nach außen offen ist. Die Längsausdehnung der Kapillarstruktur erstreckt sich von dem proximalen Ende, welches mit dem Haltebereich verbunden ist, bis zum distalen Bereich, der zum Einstechen in die Haut vorgesehen ist. Die Hohlkanülen des Standes der Technik weisen lediglich an ihrem äußersten distalen Ende eine Öffnung auf, durch die Körperflüssigkeit eintreten kann. Im Gegensatz dazu kann die erfindungsgemäße Kapillarstruktur über einen wesentlich größeren Teil der Längsausdehnung Körperflüssigkeit aufnehmen. Im Regelfall beträgt die Länge des Bereiches der Kapillarstruktur, welcher nach außen offen ist, mehr als 10 % der Längsausdehnung der Kapillarstruktur, vorzugsweise mehr als 50 % der Längsausdehnung. Herstellungstechnisch besonders günstig ist es, wenn die Kapillarstruktur entlang ihrer gesamten Längsausdehnung nach außen offen ist.

Herkömmliche Hohlkanülen werden im Stand der Technik durch Ausziehen dickerer Rohre hergestellt. Hieraus ergibt sich, dass die Herstellung von sehr dünnen Hohlkanülen, beispielsweise unterhalb von 0,3 mm Außendurchmesser sehr aufwendig und kostspielig ist. In der US 5,801,057 wird hingegen ein anderer Weg beschrieben. Aus Silizium wird ein erster Körper geätzt, der einen Nadelbereich mit einer Rinne und eine Messkammer, die mit der Nadel integral verbunden ist, besitzt Messkammer und Rinne im Nadelbereich werden darauffolgend mit einer Schicht verschlossen. Eine Verbindung der beiden Körper wird beispielsweise durch anodisches Bonden erreicht. Im Hinblick auf die starke Miniaturisierung der Blutentnahmevorrichtung und im Hinblick auf den Verfahrensschritt des Bondens treten erhebliche Herstellkosten auf. Weiterhin kann die so entstehende Anordnung, entsprechend den vorstehend bereits genannten Dokumenten des Standes der Technik ebenfalls nur Flüssigkeit über den Spitzenbereich der Nadel aufnehmen. Erfindungsgemäß wurde gefunden, dass es möglich ist, eine effiziente Aufnahme von Flüssigkeit auch zu erzielen, wenn eine offene Kapillare vorliegt. Beispiele für derartige offene Kapillare werden im folgenden beschrieben:
Offene Kapillare können analog zu dem Dokument US 5,801,057 durch Fotolithografieverfahren hergestellt werden, wie sie aus dem Bereich der Halbleitertechnologie bekannt sind. Es ist weiterhin auch möglich, Vollnadeln durch Fräsen, Ätzen und dgl. mit Rinnen, Nuten oder dgl. zu versehen, die nach außen offen sind. Derartige Vertiefungen führen von der Spitze, zumindest aus einem der Spitze benachbarten Bereich zum proximalen Ende der Nadel, welcher mit der Halterung verbunden ist. Diese Vertiefungen bzw. Kapillaren müssen nicht notwendigerweise gerade verlaufen, sondern können auch beispielsweise spiralförmig, meanderförmig oder dgl. angeordnet sein. Von Bedeutung ist es, dass durch die Kapillaren Flüssigkeit aus dem distalen Bereich der Nadel in den proximalen Bereich transportiert wird. Der Querschnitt der Kapillaren kann beispielsweise V-förmig, halbkreisförmig oder auch rechteckig sein. Entscheidend ist es, dass ein Teil des Querschnittes nach außen offen ist, so dass Flüssigkeit über die Außenumfangsfläche der Nadel in den Kapillarkanal eindringen kann.

Neben den bereits genannten Möglichkeiten, Kapillarkanäle in stabförmige Körper einzubringen, ist es auch möglich, die Kapillarkanäle durch Zusammenfügen von Körpern zu generieren. So ist es beispielsweise möglich, zwei oder mehr Vollnadeln aneinander zu befestigen, zum Beispiel aneinander zu schweißen, so dass durch die Kontaktbereiche der Vollnadeln Kapillarkanäle gebildet werden. Entsprechend ist es auch möglich, Drähte, in Form einer Litze, miteinander zu verdrillen, so dass eine Vielzahl von Kontaktbereichen, welche Kapillarkanäle generieren, gebildet werden.

Die Kapillarkanäle, welche in der Kapillarstruktur vorhanden sind, sind typischerweise tiefer als breit. Der Quotient aus Tiefe zu Breite, (im allgemeinen als Aspektverhältnis bezeichnet) liegt vorteilhaft bei 2 bis 5. Der Querschnitt des Kapillarkanales ist typischerweise größer als 2500 µm² und kleiner als 1mm². Wie bereits weiter oben ausgeführt, ist es günstig, dass die Kapillarkanäle von außen zugänglich sind, so dass auch Körperflüssigkeit in sie aufgenommen werden kann, während die Kapillarstruktur ins Gewebe eingestochen ist. Um eine gute Aufnahme von Körperflüssigkeit zu erzielen, sollte der nach außen offene Bereich der Kapillarstruktur eine Länge von 1 mm oder mehr aufweisen.

An dem proximalen Teil der Kapillarstruktur schließt sich der Haltebereich an. Haltebereich und Kapillarstruktur können sowohl einstückig (monolithisch) ausgebildet sein als auch separate Teile darstellen, die durch Kleben, Schmelzen, Presssitz oder dgl. miteinander verbunden sind. Monolithische Strukturen können besonders günstig aus Halbleitern mit den für Halbleiter bekannten Herstellungsverfahren erzeugt werden. Hierdurch kann eine sehr starke Miniaturisierung erreicht werden. Eine Herstellung der Stecheinheit aus einem separaten Haltebereich und einer separaten Kapillarstruktur kann hingegen im Hinblick auf die Herstellkosten günstiger sein. Eine Stechvorrichtung aus separaten Elementen kann beispielsweise aus einer metallischen Kapillarstruktur und einem Haltebereich aus Kunststoff gebildet werden.

Eine erfindungsgemäße Stecheinheit kann im proximalen Bereich der Kapillarstruktur oder im Haltebereich eine Auswertezone aufweisen. Wird eine Auswertung der Analytkonzentration beispielsweise durch eine Infrarotspektroskopie durchgeführt, so braucht die Nachweiszone keine weiteren Reagenzien zu enthalten, die eine Bestimmung des Analyten ermöglichen. Da die Materialien für die Kapillarstruktur und den Haltebereich in aller Regel nicht Infrarot-durchlässig sind, so wird eine reflexionsspektroskopische Analyse bevorzugt. Hierzu ist die Auswertezone vorzugsweise IR reflektiv, was bei Metalloberflächen in der Regel hinreichend gegeben ist. Kunststoffe können beispielsweise durch Aufdampfen oder Aufsputtern von Gold oder Aluminium IR reflektiv gemacht werden. (Alternativ können auch optisch transparente Fenster integriert werden.)

Im bevorzugten Fall besitzt die Stecheinheit jedoch eine Nachweiszone, in der ein Reagenz angeordnet ist, welches mit einem nachzuweisenden Analyten in der Körperflüssigkeitsprobe eine detektierbare Veränderung erfährt. Typische Reagenzien zum Nachweis von Glucose basieren beispielsweise auf Glucoseoxidase in Verbindung mit einem farbgebenden Redoxsystem. Für eine optische Auswertung sind Reagenzien, die mit Glukose aus der Körperflüssigkeit eine Farbe bilden, aus dem Stand der Technik hinlänglich bekannt. Weiterhin sind aus dem Gebiet der Blutzuckermessstreifen auch Reagenzien bekannt, die einen elektrochemischen Nachweis eines Analyten ermöglichen. Da auch derartige Nachweissysteme aus dem Stand der Technik hinlänglich bekannt sind, werden sie an dieser Stelle nicht näher beschrieben.

Die genannten Reagenziensysteme können im proximalen Bereich der Kapillarstruktur angeordnet sein, aufgrund der relativ ungünstigen Möglichkeiten die Reagenzien dort zu fixieren und auszuwerten, ist jedoch eine Anordnung der Reagenzien in dem Haltebereich bevorzugt. Um eine Benetzung der Reagenzien mit der Körperflüssigkeit zu ermöglichen, schließt sich das Reagenz entweder direkt an die Kapillarstruktur an und kann durch eigene Kapillarkräfte Körperflüssigkeit aufnehmen oder aber zwischen der Kapillarstruktur und der Nachweiszone ist eine fluidische Verbindung (z.B. Verbindungskanal, Flies, o.ä.) vorgesehen, durch die die Körperflüssigkeit aus der Kapillarstruktur in die Nachweiszone gelangen kann. Die Stecheinheit kann beispielsweise so ausgebildet sein, dass ein Kapillarkanal der Kapillarstruktur in den Haltebereich weitergeführt ist und ein Reagenz im Bereich des Haltebereiches direkt auf den dort verlaufenden Kapillarkanal aufgebracht wird. Die verwendeten Reagenzgemische liegen im Normalfall fest vor und weisen aufgrund ihrer Inhaltsstoffe (z. B. Aluminiumoxid, Kieselgur und dgl.) eine so hohe Kapillarität auf, dass sie aus dem Kapillarkanal Körperflüssigkeit aufnehmen können.

Die Form des Haltebereiches ist relativ unkritisch. Sie kann beispielsweise die Form eines kleinen Quaders aufweisen, der eine Vertiefung zur Aufnahme des Reagenzgemisches besitzt. Zur Befestigung der Stecheinheit in einer Antriebseinheit sind im Regelfall keine besonderen Maßnahmen notwendig bzw. es kann auf Gestaltungen zurückgegriffen werden, wie sie für disposible Lanzetten herkömmlicher Blutentnahmesysteme bekannt sind. Beispielsweise kann der Haltebereich Verjüngungen aufweisen, in die Federelemente einer Halterung der Antriebseinheit eingreifen, um die Stecheinheit zu haltern. Vorteilhaft wird die Stecheinheit innerhalb der Halterung so positioniert, (zum Beispiel durch Andrücken einer der Spitze der Stecheinheit abgewandten Seite an einem Anschlag), dass eine gute Kontrolle der Einstechtiefe der Stecheinheit gewährleistet werden kann. Für eine derartige Halterung und das Zusammenwirken von Halterung und disposibler Stecheinheit wird auf das Dokument EP B 0 565 970 Bezug genommen.

In ein System zur Entnahme kleiner Körperflüssigkeitsmengen kann vorteilhaft eine Detektionseinheit integriert sein. Wird eine Stecheinheit mit einem Reagenz verwendet, das bei Anwesenheit eines Analyten eine Farbveränderung bzw. Farbbildung durchläuft, so kann das System eine optische Detektionseinheit, beinhaltend eine Lichtquelle und einen Detektor zur Detektion von transmittiertem oder reflektiertem Licht aufweisen. Bei Verwendung einer elektrochemischen Detektion kann das System Elektroden aufweisen, die das Reagenz der Stecheinheit bzw. die Kontakte der Stecheinheit kontaktieren, welche ihrerseits das Reagenz kontaktieren. Zur Auswertung kann das System die im Stand der Technik bekannten elektronischen Einrichtungen besitzen, um die Konzentration des Analyten beispielsweise durch Messung des sog. Cotrell-Stromes zu ermitteln. Für den Fall, dass eine reagenzfreie Analyse durchgeführt werden soll, so kann das System beispielsweise eine Infrarotstrahlungsquelle und einen Infrarotdetektor, sowie Einrichtungen zur spektralen Auflösung der reflektierten Strahlung der Auswertezone beinhalten.

Das erfindungsgemäße Entnahmesystem weist weiterhin eine Antriebseinheit auf, die die Halterung bei Aktivierung von einer ersten in eine zweite Position verfährt, so dass die Stecheinheit eine Stechbewegung ausführt. Derartige Antriebseinheiten sind aus dem Bereich der Blutentnahmesysteme hinlänglich bekannt. Sie können beispielsweise eine Feder beinhalten, die vom Benutzer gespannt wird und die beim Entspannen die Stecheinheit antreibt. Eine besonders vorteilhafte Antriebseinheit ist in der EP B 0 565 970 beschrieben.

Mit der erfindungsgemäßen Stecheinheit bzw. dem erfindungsgemäßen Entnahmesystem kann eine Entnahme von Körperflüssigkeit sowohl erfolgen, während die Kapillarstruktur bzw. ein Teil davon in die Haut eingestochen ist (d.h. Entnahme direkt aus dem Körper oder von aus dem Körper an die Körperoberfläche austretender Körperflüssigkeit)oder aber die Kapillarstruktur kann nach Ausführen eines Stiches aus dem Körper zurückgezogen werden und von der Körperoberfläche austretende Körperflüssigkeit aufnehmen. Eine Entnahme, bei der die Kapillarstruktur zur Aufnahme von Körperflüssigkeit in dem Körper verbleibt, ist insbesondere für eine Probennahme am Arm geeignet. Dies liegt darin begründet, dass sich kleine Stichwunden am Arm sehr schnell wieder schließen, so dass nach dem Stich keine oder nur sehr kleine Flüssigkeitsmengen austreten. Andererseits ist am Arm das Schmerzempfinden geringer ausgeprägt als z. B. am Finger, so dass ein Verbleiben der Kapillarstruktur im Körper nicht als schmerzhaft empfunden wird. Wie bereits weiter oben ausgeführt, weist eine nach außen offene Kapillarstruktur gegenüber den herkömmlichen Hohlkanülen den Vorteil auf, dass über den offenen Bereich Flüssigkeit aufgenommen werden kann, während der Bereich der Flüssigkeitsaufnahme bei Hohlkanülen lediglich auf das vordere Ende der Kanüle beschränkt ist. Letzteres ist insbesondere nachteilig, wenn sich die Nadelöffnung beim Einstich durch Gewebeteile verschließt, so dass keine oder nicht ausreichend Flüssigkeit aufgenommen werden kann.

Auch bei einer Entnahme, bei der die Kapillarstruktur nach dem Stechvorgang aus dem Gewebe zurückgezogen wird, besitzt sie gegenüber den herkömmlichen Hohlkanülen einen Vorteil. Wie bereits ausgeführt, lassen sich nach außen offene Kapillarstrukturen einfacher und kostengünstiger herstellen als geschlossene Hohlkanülen.

Mit den erfindungsgemäßen Stecheinheiten kann weiterhin ein Entnahmevorgang durchgeführt werden, der eine Kombination aus den vorstehend genannten Verfahren ist. Bei diesem Kombinationsverfahren wird zunächst eingestochen, die Kapillarstruktur um einen Teil der Einstichstrecke zurückgezogen und dort für eine Sammelperiode von einigen Sekunden verharren gelassen. Dieses Verfahren weist den Vorteil auf, dass durch das Zurückziehen der Kapillarstruktur ein Teil des Stichkanales freigegeben wird, so dass sich Körperflüssigkeit in ihm sammeln kann und von dort in die Kapillarstruktur eindringt.

Ein weiterer entscheidender Faktor, der wichtig für eine effiziente Aufnahme von Körperflüssigkeit mittels der Kapillarstruktur ist, stellt die Benetzbarkeit der Kapillarkanäle dar. Werden Kapillarstrukturen aus Silizium verwendet, so sind diese in der Regel bereits durch eine Siliziumoxidschicht auf der Oberfläche ausreichend benetzbar. Werden für die Kapillarstruktur Metalle verwendet, so sind diese häufig relativ schlecht benetzbar. Dem kann jedoch durch eine Reihe geeigneter Maßnahmen, wie z. B. durch eine Silikatisierung der Oberfläche entgegengewirkt werden. Eine ausreichende hohe Benetzbarkeit ist im Regelfall dann gegeben, wenn die Flüssigkeit in den Kapillaren einen konkaven Meniskus aufweist bzw. was gleichbedeutend ist, wenn der Benetzungswinkel kleiner als 90 ° ist.

Die Erfindung wird anhand von Figuren näher dargestellt:
- Figur 1:: Disposible Stecheinheit aus Silizium
- Figur 2:: System zur Entnahme von Körperflüssigkeit beinhaltend die Stecheinheit aus Figur 1 und eine Antriebseinheit, sowie eine optische Auswertevorrichtung
- Figur 3:: Kapillarbereich einer disposiblen Stecheinheit gebildet aus 2 miteinander ver- schweißten Vollnadeln.
- Figur 4:: Kapillarstruktur gebildet aus miteinander verdrillten Drähten (Litzenstruktur)

- Figur 5:: Kapillarstruktur in Form einer Vollnadel auf deren Oberfläche sich Kapillarkanäle befinden.

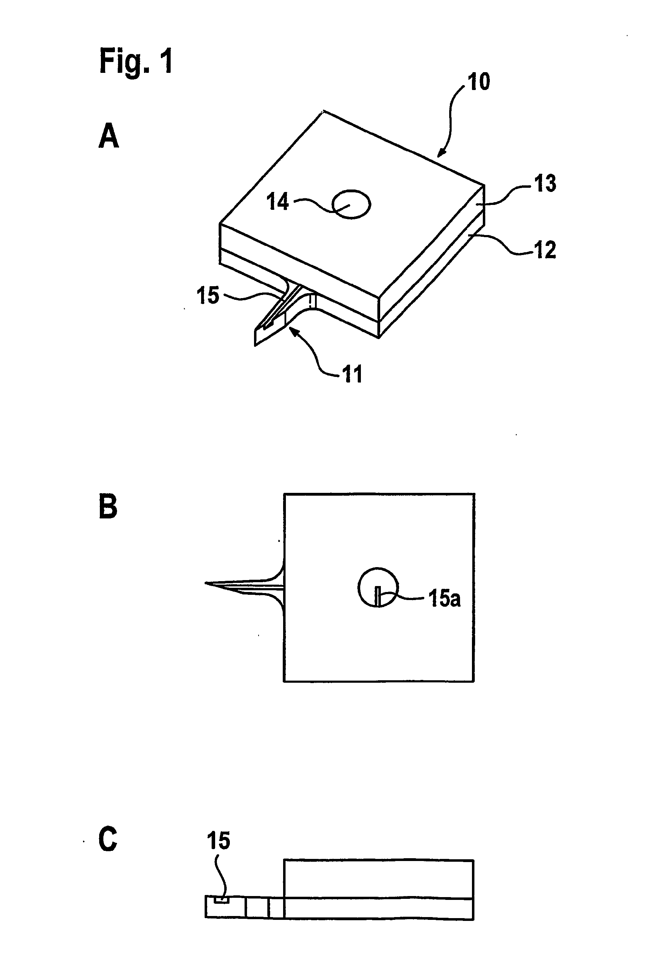

Fig. 1 zeigt eine disposible Stecheinheit in drei Ansichten. Aus der perspektivischen Ansicht in Fig. 1A ist zu erkennen, dass die Stecheinheit 10 eine Kapillarstruktur 11 besitzt, welche in einem Haltebereich 12 angeordnet ist. Der Haltebereich 12 ist mit einer Platte 13 gedeckelt, welche ein Fenster 14 aufweist. Die Kapillarstruktur 11 ist so ausgebildet, dass sie an ihrem distalen Ende eine Spitze aufweist, um Haut zu durchstechen. Weiterhin ist in der Kapillarstruktur ein Kapillarkanal 15 angeordnet, welcher nach oben offen ist. Dieser Kapillarkanal verläuft im Inneren der Stecheinheit weiter und gelangt zu einer Nachweiszone, die unterhalb des Fensters 14 angeordnet ist. In Fig. 1B ist das Ende 15 a des Kapillarkanals im Fensterbereich zu erkennen. Das Ende ist in diesem Fall sichtbar, da oberhalb des Kanals keine Testchemie angeordnet ist. Im Einsatzzustand wird jedoch oberhalb dieses Endes ein Nachweisbereich, z. B. ein optisches Detektionssystem für Glukose angeordnet.

Fig. 1C zeigt eine Seitenansicht der Stecheinheit, aus welcher erkennbar ist, dass der Kapillarkanal 15 sowohl nach oben als auch am distalen Ende der Kapillarstruktur seitlich offen ist. Die Länge der dargestellten Kapillarstruktur beträgt 1.6 mm und Breite und Tiefe des Kapillarkanals 15 betragen 50 bzw. 150 µm.

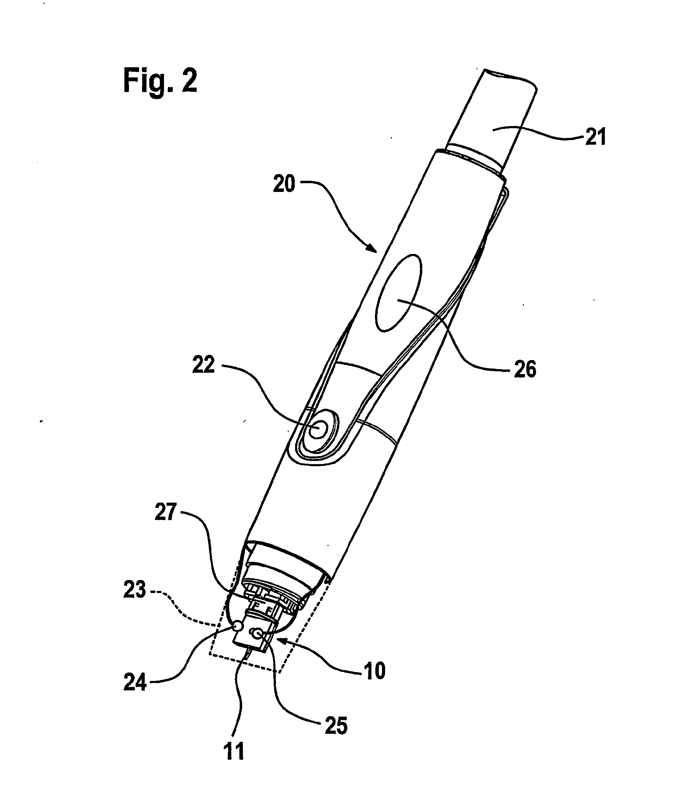

Fig. 2 zeigt ein System (20) zur Entnahme von Körperflüssigkeit, welches eine Stecheinheit gemäß Fig. 1 sowie eine Antriebseinheit und eine optische Auswertevorrichtung beinhaltet. Die Figur zeigt ein System, welches auf einer Blutentnahmevorrichtung gemäß EP 1034740 basiert. Die Stecheinheit gemäß Fig.1 befindet sich in der Halterung der Blutentnahmevorrichtung, in der bei bisherigen Systemen eine disposible Lanzette angeordnet ist. Durch Betätigung des Druckknopfes 21 wird der Antriebsmechanismus gespannt und durch Betätigung des Auslöseknopfes 22 wird der Stechvorgang, d. h. die Bewegung der Stecheinheit ausgelöst. Hierbei tritt der Kapillarbereich 11 aus einer Austrittsöffnung (nicht dargestellt) der Kappe 23 (gestrichelt dargestellt) aus und punktiert Haut, welche sich zur Blutentnahme an der Kappenöffnung befindet. Im Gegensatz zu den Blutlanzettenvorrichtungen, welche unter der Bezeichnung Softclix Pro im Handel sind, wird der Kapillarbereich jedoch nicht hinter die Kappenöffnung zurückgezogen, sondern verbleibt mit seiner maximalen Einstechtiefe im Gewebe oder wird partiell zurückgezogen, so dass eine Aufnahme von austretender Körperflüssigkeit in die Kapillare möglich ist. Wie im Zusammenhang mit Fig. 1 geschildert, gelangt Körperflüssigkeit durch die Kapillare in einen Auswertebereich, in dem eine Analytbestimmung möglich ist. In Fig. 2 ist ein optisches Auswertesystem dargestellt, welches eine Lichtquelle 24 sowie ein Fotodetektor 25 beinhaltet. Mit der Lichtquelle 24 (z. B. eine Leuchtdiode) wird durch das Fenster 14 der Auswertebereich beleuchtet und von dem Auswertebereich reflektierte Strahlung mit dem Fotodetektor 25 aufgefangen. Mit einer in dem System angeordneten Auswerteeinheit (nicht dargestellt) wird aus der mit dem Fotodetektor empfangenen Intensität eine Analytkonzentration ermittelt und auf dem Display 26 dargestellt. Nach erfolgter Messung kann der Benutzer das System 20 von der Körperoberfläche entfernen, die Kappe 23 abnehmen und die Stecheinheit 10 auswerfen. Für eine weitere Messung kann eine neue Stecheinheit, z. B. aus einem Magazin, in die Halterung 27 des Systems aufgenommen werden.

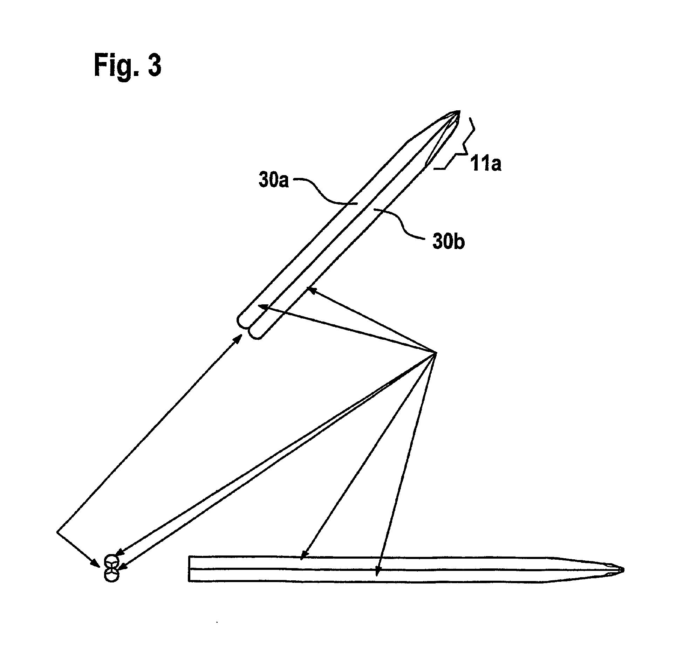

Fig. 3 zeigt die Generierung einer Kapillarstruktur durch Verschweißung zweier Vollnadeln. In Fig. 3 ist zu erkennen, dass durch Verschweißung zweier zylindrischer Metalldrähte in dem Verbindungsbereich zwei gegenüberliegende Kapillarkanäle gebildet werden, die nach außen offen sind. Die erhaltene Struktur wird an einem Ende angeschliffen, um eine Spitze zu bilden, die ein Einstechen in Haut erlaubt. Im dargestellten Beispiel wurden Metalldrähte aus medizinischem Edelstahl mit einem Querschnitt von 400 µm verwendet. Der Spitzenbereich 11a der Kapillarstruktur weist eine Länge von etwa 2 mm auf. Das Verschweißen der beiden Drähte erfolgte durch Hindurchleiten von Strom durch die beiden Drähte, wobei der eine Draht als Anode und der andere Draht als Kathode geschaltet wurde.

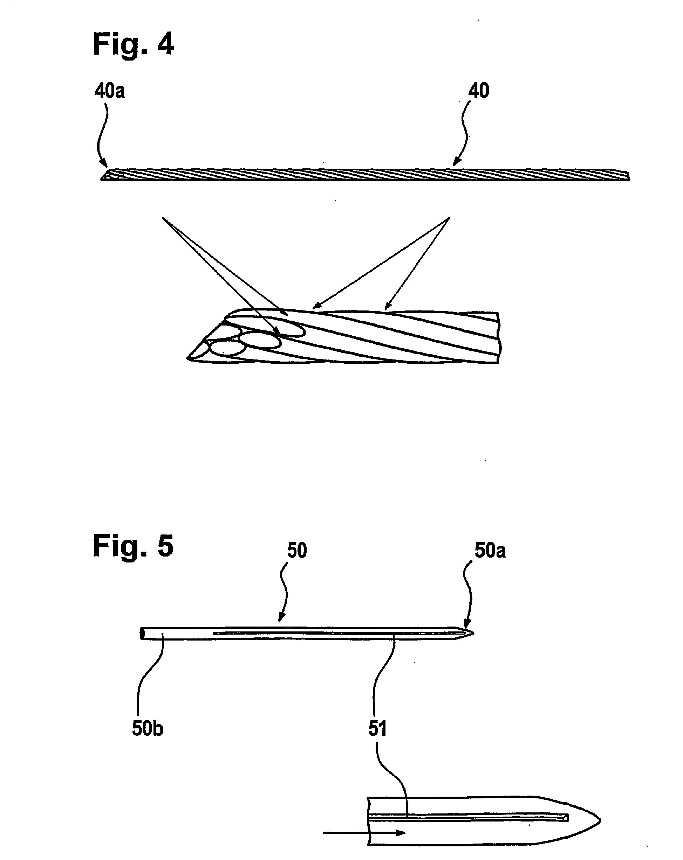

Fig. 4 zeigt eine Kapillarstruktur in Form einer Litze. Zur Generierung dieser Litzenstruktur 40 wurden Metalldrähte mit einem Durchmesser von 20 bis 70 µm miteinander verdrillt und an dem einen Ende schräg abgeschnitten, so dass sich ein Spitzenbereich 40a ergibt, welcher in die Haut einstechbar ist. Die Ausschnittvergrößerung in Fig. 4, welche den Spitzenbereich zeigt, lässt die durch die nebeneinander liegenden Drähte entstehenden, offenen Kapillarkanäle (durch Pfeile gekennzeichnet) erkennen.

In Fig. 5 ist eine Kapillarstruktur 50 dargestellt, welche aus einer Vollnadel generiert wurde. Die Nadel weist einen proximalen Bereich 50b auf, welcher in einem Haltebereich gehaltert werden kann. Der Spitzenbereich 50 A der Nadel ist wie bei konventionellen Blutlanzetten angeschliffen, um ein einfaches, schmerzarmes Eindringen in Haut zu ermöglichen. In die Vollnadel wurde durch Fräsen ein Kapillarkanal 51 eingebracht, welcher nach außen offen ist. Der Querschnitt dieses Kapillarkanals weist etwa 60 x 150 µm auf.

## Patentansprüche

1. System (20) zur Entnahme kleiner Körperflüssigkeitsmengen mit
einer Antriebseinheit, die eine Halterung (27) aufweist, welche bei Aktivierung der Antriebseinheit von einer ersten in eine zweite Position verfährt, sowie mit einer disposiblen Stecheinheit (10), die einen Haltebereich (12) aufweist, welcher entnehmbar in der Halterung angeordnet ist, wobei mit dem Haltebereich das proximale Ende einer länglichen Kapillarstruktur (11) mit mindestens einem Kapillarkanal (15) zum Transport von Körperflüssigkeit verbunden ist, wobei der Transport in Richtung des proximalen Endes der Kapillarstruktur in Folge von Kapillarkräften erfolgt, und das distale Ende der Kapillarstruktur eine Spitze besitzt, die zum Durchstechen von Haut geeignet ist, und
wobei sich das distale Ende der Kapillarstruktur bei Anordnung der Halterung in der ersten Position außerhalb der Haut befindet und in der zweiten Position bis zu einer Tiefe, der Einstechtiefe, in die Haut eingedrungen ist, **dadurch gekennzeichnet, dass**
der mindestens eine Kapillarkanal in einem Bereich, der an der Spitze der Kapillarstruktur beginnt und sich über die Spitze hinaus erstreckt, entlang der Längsausdehnung der Kapillarstruktur nach außen offen ist, und die Form einer Rinne aufweist.

2. System gemäß Anspruch 1, bei dem die gesamte Länge der Kapillarstruktur vom proximalen bis zum distalen Ende nach außen offen ist.

3. System gemäß Anspruch 1, bei dem der Haltebereich eine Nachweiszone zum Nachweis eines oder mehrerer Analyten aufweist, wobei die Nachweiszone so angeordnet ist, dass sie Körperflüssigkeit von der Kapillarstruktur aufnehmen kann.

4. System gemäß Anspruch 1, bei dem die Antriebseinheit so ausgebildet ist, dass sie die Stecheinheit so verfährt, dass diese in der zweiten Position für ein Zeitintervall (Sammelperiode), verharrt und die Stecheinheit darauf in eine Position verfahren wird, bei der sich das distale Ende der Kapillarstruktur außerhalb der Haut befindet.

5. System gemäß Anspruch 1, bei dem die Antriebseinheit so ausgebildet ist, dass sie die Stecheinheit so verfährt, dass diese nach Erreichen der zweiten Position in eine Sammelposition zurückbewegt wird, in der das in der Haut befindliche Teilstück der Kapillarstruktur kürzer ist als in der zweiten Position.

6. System gemäß Anspruch 1, bei dem Kapillarstruktur und Haltebereich integral miteinander verbunden sind.

7. System gemäß Anspruch 1 oder 6, bei dem Haltebereich und Kapillarstruktur aus einem Halbleiter, vorzugsweise Silizium hergestellt sind.

8. System gemäß Anspruch 6 oder 7, bei dem Haltebereich und Kapillarstruktur integral aus einem einzelnen Materialteil gefertigt sind.

9. System gemäß Anspruch 1, bei dem der rinnenförmige Bereich einen im wesentlichen V-förmigen Querschnitt aufweist.

10. System gemäß Anspruch 1, bei dem die Länge der Kapillarstruktur im Bereich von 0,3 bis 3 mm und der Querschnitt der Kapillarstruktur im Bereich von 0,03 bis 0,8 mm liegt.

11. Disposible Stecheinheit (10) zur Entnahme kleiner Körperflüssigkeitsmengen, die einen Haltebereich (12) aufweist, mit dem das proximale Ende einer länglichen Kapillarstruktur mit mindestens einem Kapillarkanal (15) zum Transport von Körperflüssigkeit verbunden ist, wobei der Transport in Richtung des proximalen Endes der Kapillarstruktur in Folge von Kapillar-Kräften erfolgt, und das distale Ende der Kapillarstruktur eine Spitze besitzt, die geeignet ist Haut zu durchstechen, sowie der mindestens eine Kapillarkanal in einem Bereich, der an der Spitze der Kapillarstruktur beginnt und sich über die Spitze hinaus erstreckt, entlang der Längsausrichtung der Kapillarstruktur, nach außen offen ist und die Form einer Rinne aufweist.

## Claims

1. System (20) for withdrawing small amounts of body fluid comprising:
a drive unit which has a holder (27) which is moved from a first position into a second position when the drive unit is activated, as well as a disposable lancing device (10) which has a holding area (12) that is removably positioned in the holder, wherein the proximal end of an elongate capillary structure (11) comprising at least one capillary channel (15) for transporting body fluid is connected to the holding area, said transport taking place in the direction of the proximal end of the capillary structure as a result of capillary forces, and the distal end of the capillary structure has a tip that is suitable for piercing skin and
wherein the distal end of the capillary structure is located outside the skin when the holder is arranged in said first position and in the said second position is inserted into the skin up to a depth, the puncture depth, **characterized in that**
the at least one capillary channel is open to the outside in an area along the longitudinal extension of the capillary structure, starting at the tip of the capillary structure and extending beyond the tip, and has a channel shape.

2. System according to claim 1, in which the entire length of the capillary structure from the proximal to the distal end is open to the outside.

3. System according to claim 1, in which the holding area has a detection zone for detecting one or several analytes, the detection zone being arranged such that the detection zone can take up body fluid from the capillary structure.

4. System according to claim 1, in which the drive unit is designed to move the lancing unit in such a manner that the lancing unit remains in the second position for a time interval (collection period) and subsequently the lancing unit is moved into a position in which the distal end of the capillary structure is outside the skin.

5. System according to claim 1, in which the drive unit is designed to move the lancing unit in such a manner that after reaching the second position the lancing unit is moved back into a collecting position in which the section of the capillary structure located in the skin is shorter than in the second position.

6. System according to claim 1, in which the capillary structure and holding area are integrally connected together.

7. System according to claim 1 or 6, in which the holding area and capillary structure are manufactured from a semiconductor, preferably silicon.

8. System according to claim 6 or 7, in which the holding area and capillary structure are integrally manufactured from a single piece of material.

9. System according to claim 1, in which the channel-shaped area has an essentially V-shaped cross-section.

10. System according to claim 1, in which the length of the capillary structure is in the range of 0.3 to 3 mm and the cross-section of the capillary structure is in the range of 0.03 to 0.8 mm.

11. Disposable lancing unit (10) for withdrawing small amounts of body fluids which has a holding area (12) which is connected to the proximal end of an elongate capillary structure comprising at least one capillary channel (15) for transporting body fluid, said transport taking place in the direction of the proximal end of the capillary structure as a result of capillary forces, and the distal end of the capillary structure has a tip which is suitable for piercing skin and which has at least one capillary channel that is open to the outside and has a channel shape in an area along the longitudinal extension of the capillary structure which starts at the tip of the capillary structure and extends beyond the tip.

## Revendications

1. Système (20) de prélèvement de petites quantités de liquide organique comprenant
une unité d'entraînement, qui présente un support (27), lequel se déplace d'une première dans une seconde position en cas d'activation de l'unité d'entraînement, et une unité de perforation (10) jetable, qui présente une zone de retenue (12), laquelle est disposée de façon amovible dans le support, l'extrémité proximale d'une structure capillaire (11) allongée présentant au moins un canal capillaire (15) étant reliée à la zone de retenue pour le transport de liquide corporel, le transport s'effectuant en direction de l'extrémité proximale de la structure capillaire sous l'effet de forces capillaires, et l'extrémité distale de la structure capillaire présentant une pointe, qui convient pour la perforation de la peau, et
l'extrémité distale de la structure capillaire se trouvant à l'extérieur de la peau en cas d'agencement du support dans la première position et étant pénétrée dans la peau en cas d'agencement du support dans la seconde position jusqu'à une profondeur, la profondeur de perforation, **caractérisé en ce que**
l'au moins un canal capillaire est ouvert vers l'extérieur dans une zone, qui commence sur la pointe de la structure capillaire et s'étend au-delà de la pointe, le long de l'extension longitudinale de la structure capillaire, et présente la forme d'une rigole.

2. Système selon la revendication 1, sur lequel l'ensemble de la longueur de la structure capillaire est ouvert vers l'extérieur depuis l'extrémité proximale jusqu'à l'extrémité distale.

3. Système selon la revendication 1, sur lequel la zone de retenue présente une zone de décèlement pour le décèlement d'un ou de plusieurs analytes, la zone de décèlement étant disposée de telle sorte qu'elle peut recevoir du liquide corporel de la structure capillaire.

4. Système selon la revendication 1, sur lequel l'unité d'entraînement est conçue de telle sorte qu'elle déplace l'unité de perforation de façon que celle-ci reste dans la seconde position pour un intervalle de temps (période de collecte) et l'unité de perforation est déplacée ensuite dans une position dans laquelle l'extrémité distale de la structure capillaire se trouve à l'extérieur de la peau.

5. Système selon la revendication 1, sur lequel l'unité d'entraînement est conçue de telle sorte qu'elle déplace l'unité de perforation de façon que celle-ci soit déplacée en arrière après avoir atteint la seconde position dans une position de collecte dans laquelle le tronçon, se trouvant dans la peau, de la structure capillaire est plus court que dans la seconde position.

6. Système selon la revendication 1, sur lequel la structure capillaire et la zone de retenue sont reliées l'une à l'autre de façon intégrale.

7. Système selon la revendication 1 ou 6, sur lequel la zone de retenue et la structure capillaire sont fabriquées à partir d'un semi-conducteur, de préférence du silicium.

8. Système selon la revendication 6 ou 7, sur lequel la zone de retenue et la structure capillaire sont fabriquées de façon intégrale à partir d'une partie de matériau individuelle.

9. Système selon la revendication 1, sur lequel la zone en forme de rigole présente une section sensiblement en forme de V.

10. Système selon la revendication 1, sur lequel la longueur de la structure capillaire se situe dans la plage de 0,3 à 3 mm et la section de la structure capillaire dans la plage de 0,03 à 0,8 mm.

11. Unité de perforation (10) jetable pour le prélèvement de petites quantités de liquide corporel, qui présente une zone de retenue (12), à laquelle l'extrémité proximale d'une structure capillaire allongée présentant au moins un canal capillaire (15) est reliée pour le transport de liquide corporel, le transport s'effectuant en direction de l'extrémité proximale de la structure capillaire sous l'effet de forces capillaires, et l'extrémité distale de la structure capillaire présentant une pointe qui convient pour perforer la peau et l'au moins un canal capillaire étant ouvert vers l'extérieur dans une zone, qui démarre à la pointe de la structure capillaire et s'étend au-delà de la pointe, le long de l'extension longitudinale de la structure capillaire, et présente la forme d'une rigole.
